# EUROPEAN PATENT APPLICATION

(11) **EP 3 944 812 A1**
(43) Date of publication of application: **02.02.2022**
(21) Application number: 21150968.2
(22) Date of filing: 11.01.2021
(51) Int. Cl.: A61B 5/11, A61B 5/16, A61B 5/00

(54) **DEVICE AND METHOD FOR THE PREVENTION AND TREATMENT OF OBSTRUCTIVE SLEEP APNEA SYNDROME**

(30) Priority: 27.07.2020 IT 202000018184
(71) Applicant: Beon Solutions S.r.l., 31059 Zero Branco (TV) (IT)
(72) Inventor: Florian, Alessandro, 31059 Zero Branco (IT)
(74) Representative: Leganza, Alessandro

(57) **Abstract**

A diagnostic device and method to treat obstructive sleep apnea syndrome by analysing the vestibule-ocular reflex, both for the prevention phase and for the treatment of obstructive sleep apnea syndrome, comprises a head rotational acceleration measuring unit, and a display unit to display moving letters.

## Description

### Technical field

The present invention relates to a device to treat obstructive sleep apnea syndrome, both for the prevention phase and for the treatment of obstructive sleep apnea syndrome. It also concerns a diagnostic method for identifying patients potentially suitable for developing obstructive sleep apnea syndrome

### Background of the invention

Obstructive Sleep Apnea Syndrome or Obstructive Sleep Apnea Syndrome, also known as OSAS (Obstructive Sleep Apnea Syndrome), is a condition characterized by pauses in breathing during sleep, due to partial or total obstruction of the upper airways. In the first case, hypopnea is determined, that is, passage of air flow in the airways is reduced, in the second case, apnea, that is, the temporary suspension of respiratory movements associated with the complete interruption of the air flow, for a time more than 15 second.

Obstructive sleep apnea (OSA) is a common disorder characterized by repetitive episodes of interruption of nocturnal breathing due to the collapse of the upper airways. OSA causes severe symptoms, such as an excessive daytime sleepiness, and is associated with significant cardiovascular morbidity and mortality.

The treatment of obstructive sleep apnea syndrome is now achieved through several options, in particular, after more than three decades from its first use, continuous positive airway pressure (CPAP) is still recognized as the standard treatment. Mandibular advancement devices, in particular, when custom made, are effective from mildly to moderately into OSA and provide a possible alternative for patients intolerant to CPAP therapy. The role of surgery to treat OSAS remains controversial.

Vision plays a key role in the knowledge of our environment during each moment of our day life. It is essential for the coordination of all our movements in natural space, for the precision to perform a task, to use a tool, for reading or writing and it is also fundamental in relationships and social communication with other humans. It is known that the stabilization of an image on the retina depends on the activity of the vestibular and visual systems. The activity of each of these two systems depends on the oscillation frequency of the head. For example, at low frequencies (<0.1 Hz) the visual system is predominant, at medium frequencies, the vestibular and visual systems interact together to stabilize the gaze while at high frequencies (from 1 to 5 Hz), only the vestibular system enters into action.

When the head rotates rapidly in the horizontal or in the anterior or posterior planes, the two semi-circular channels of each pair participate in the estimation of how fast the head movement. The ipsilateral canal at rotation provides the main excitatory information - via the vestibular nerve fibers - to which the weaker and more inhibiting information from the contra-lateral channel is added. In response, the oculomotor system will induce eye movement equal in amplitude but in a direction opposite to the movement of the head. This reflex, called the vestibulo-ocular reflex (abbreviated VOR), allows the image stabilization on the retina during rapid movements of the head and is estimated during a test known as the functional Head Impulse Test (fHIT) (Halmagyi, GM, and Curthoy, IS (1988). A clinical sign of canal paresis. Arch. Neurol. 45, 737-739).

The VOR is controlled at the central Ivel (Leigh, RG, Zee, DS (2015). The Neurology of Eye Movements, 5th edition. Universit Press, Oxford) and specifically the cerebellum has a pivotal role in monitoring and adapting continuously visual gaze. For example, combining lesions and physiological studies, the cerebellar regions closely related to ocular motor functions (VOR, the pursuit and saccadic eye movements). Neurons in the vestibule-cerebellum (i.e. a region of the cerebellum found in the flocculonodular lobe that receives vestibular and visual information) are activated in relation to head or target motion, during eye fixation and vestibular responses to head motion. Those located in the nodulus and the ventral uvula modulate the velocity storage mechanism within the vestibular nuclei.

The functional Head Impulse Test (fHIT) has been developed to estimate the VOR during passive head impulses. Halmagyi et Curthoys disclosed in 1988 the "head impulse test", which allows to identify the complete loss of peripheral vestibular function (see, for general reference, Halmagyi GM, Curthoys IS. A clinical sign of canal paresis. Arch Neurol. 1988; 45: 737-739).

The rotation of the head can achieve angular accelerations of thousands of degrees per squared second.

In other words, the VOR is tested by asking to the subject to identify an optotype briefly presented during passive or active head impulse (Corallo G, Versino M, Mandala M, Colnaghi S, Ramat S. The functional head impulse test: preliminary data. J Neurol. 2018 Oct; 265 (Suppl 1): 35-39. Versino M, Colnaghi S, Corallo G, Mandala M, Ramat S. The functional head impulse test: Comparing gain and percentage of correct answers. Prog Brain Res. 2019; 248: 241-248).

Nowadays, according to the applicant's knowledge, it is not known whether the VOR may be correlated with obstructive sleep apnea syndrome.

The "head impulse test" is based on said second law of Ewald and allows to identify a pathological state even when adapting processes have occurred. While it was has been conceived in order to investigate the functionality of the horizontal semicircular canals, then it has been applied to the other semicircular canals pairs as well.

Rehabilitation for obstructive sleep apnea syndrome nowadays only partially solves the problem and in any case forces people who suffer from it to wear devices during sleep to keep the airways in continuous positive pressure.

### Summary of the invention

The problem addressed by the present invention is therefore that of providing a device that may be effectively used in the treatment of obstructive sleep apnea syndrome (OSAS). In particular, said device should be used both in the prevention phase of obstructive sleep apnea syndrome and in the treatment of obstructive sleep apnea syndrome, using a data coming from said device which serves to set up the prevention or treatment of obstructive sleep apnea syndrome.

Finally, a further problem is the development of a diagnostic method that allows to identify of patients potentially suitable for developing obstructive sleep apnea syndrome.

It has now developed an apparatus and a method which allow to treat obstructive sleep apnea syndrome, both in the preventive and/or rehabilitative phase; a diagnostic method to identify patients potentially able to develop obstructive sleep apnea syndrome, as defined in the attached claims, whose definitions are integral part of the present description, has been identified.

Further characteristics and advantages of the medical use of the apparatus of the invention and the diagnostic method of obstructive sleep apnea syndrome will result from the description of the examples of realization of the invention, provided as an indication of the invention.

### Brief description of the figures

FIGURE. 1 shows an embodiment of the invention applied to the treatment of obstructive sleep apnea syndrome.
FIGURE. 2 shows the embodiment of the invention, and highlights the (angular) movement of the head.

### Detailed description of the invention

In the present invention and unless otherwise provided, the following definitions are considered.

"Angular acceleration" is defined as the rate of change in angular velocity over time and may be expressed as degrees or radians per squared second (° / s² or rad / s²). "Passive movements" relate to the movements imposed on the patient's head by the clinician, for example, bringing the patient's head between the hands posed on the cranial-parietal bones and turning the head to some extents to the left or the right around its vertical axis . Consequently, the patient cannot predict direction, speed nor the angle of the rotation determined by said motion.

"Active rotation" refers to the movement made actively by the patients themselves by rotating the head.

"Passive rotation" refers to the movement made passively by a person to the patient himself by rotating the head.

"Loss" must be intended as the complete loss of sensitivity of an SCC, which leads to the patient being completely deprived of vestibular rotational information and therefore to the inability to properly stabilize the space and to the impairment of balance.

"Hypofunctionality or deficit", indeed, relates to any little or severe damage to the gaze stabilization and balance systems caused by deficits in the vestibular apparatus. Damages may occur because of infections, inflammatory events, pathologies, pharmacologic treatment side-effects, accidents or even traumas.

The "optotypic line" refers to an array of letters on an optotype chart used for assessing the visual acuity of a patient, such as the Snellen chart, the E.

Graph, Landolt C graph or Golovin-Sivtsev graph (for a general reference, see, for example, Ricci F. Cerulli L.: "Standardizzare dei criteri di valutazione dell'acutezza visiva." Proceedings I; Congress of the Italian Society of Legal Medicine Oculisticas, pages 35-51 Mattioli Ed. 1994).

"Visual target" refers to any image, such as drawings, symbols, numbers or letters of different sizes, that the patient is required to recognize and read.

A "Sloan letter" refers to a visual target selected from the capital letters S, O, C, D, K, V, R, H, N or Z.

"Rotational angle" is defined as the angle lying on the transverse plane and comprised between the position of a fixed point of the patient's head at the starting position and the position of the same point achieved after the rotation has been imposed. Said angle may be expressed in degrees or in radians. When rotational motions are manually produced, a certain degree of error may occur, such as ±5° with respect to the rotational angle declared.

An object of the present invention is a device for the treatment of obstructive sleep apnea syndrome comprising:
- a displaying unit (1);
- a head rotational acceleration measuring unit (2); and
- a recording unit (3).

It has in fact been surprisingly found that this device may be effectively used to prevent the onset of obstructive sleep apnea syndrome or in the treatment of obstructive sleep apnea syndrome.

The displaying unit (1), which shows the letter to be recognized by the patient, is placed at a suitable distance, for example between 30 and 350 cm, or about 150 centimeters, in front of the patient, according to the standard clinical procedures.

The displaying unit (1) may be any means suitable for showing a visual target for a period of time on the order of a few milliseconds. For example, it may be a screen on which the visual targets are displayed or projected, or it may be a computer monitor.

In a preferred non-limiting example, a computer software may govern the selection of the visual target to be displayed, which will be among those above described.

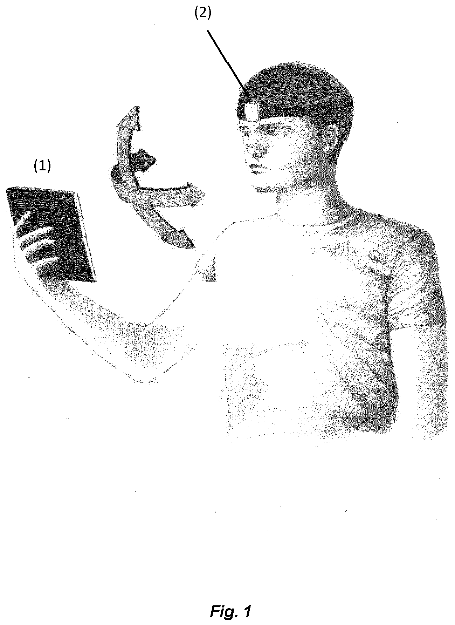

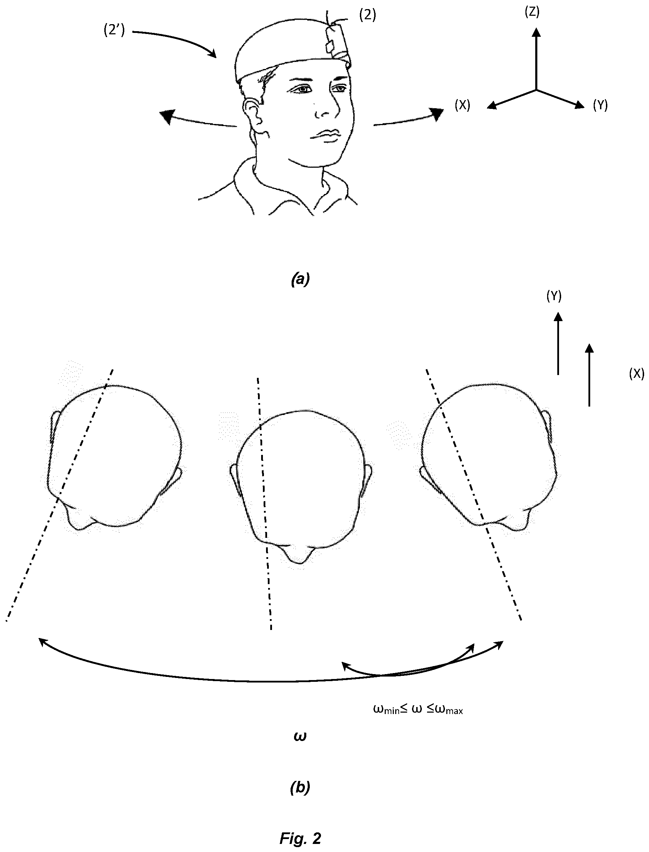

The rotational acceleration measurement unit (2) is a device capable of measuring the angular acceleration, in particular, determined by the rotation imparted to the patient's head by the clinician. Consequently, a gyroscope and/or accelerometer may be used, which provide the advantage of being easy to handle. The rotational acceleration measurement unit (2) shall be placed in such a way that it rotates contextually (integrally) with the patient's head, so that they move together. For example, a helmet or a band may be provided wherein the gyroscope (see, for example, figure 1 or 2) and/or the accelerometer are attached to it. In the case of 2 accelerometers, they need to be placed on opposite sides of the rotation axis (for example, one on the forehead and the other on the nape to measure angular accelerations in the horizontal plane). The measured value of the angular acceleration may be acquired by the device itself or, in an alternative embodiment, said unit (2) may be connected directly to a recording unit (3), directly, which is capable of recording and storing the rotational acceleration data introduced or directly acquired by the unit (2).

The head rotational acceleration measuring unit (2) placed in the patient's head has been equipped with a mode of communication to the other parts of the device which provides for a calculation to prepare the correct test and examination. It calculates the correct head movement in terms of acceleration peak and velocity peak providing the displaying unit with information on velocity and transmission time.

The device intended as a set of components displaying unit (1) (Reader), the head rotational acceleration measuring unit (2) (Sensor) and recording unit (3), is equipped with an algorithm such as to allow that the display time on the displaying unit is always correct for a specific exam, regardless of the variables and the transmission times that may vary. The times may vary in relation to variables such as useful times for the displaying unit or different processing times. The device for the diagnosis of obstructive sleep apnea syndrome recalibrates the display time data in order to allow it to be viewed by a healthy subject more times than a pathological subject. By detecting the normality detected in the scientific researches that used the device in question, it has been found that a certain time resulted in a positive response to the visualization equal to 85% by healthy people and reports this velocity on the diagnosis and screening unit by ensuring that it is correctly calibrated with respect to the main detection unit itself.

The head rotational acceleration measuring unit (2) may be an optical reader or a very high-speed camera which detects the movement of the head.

The head rotational acceleration measuring unit (2) may be a head movement optical reader device which calculates its angular velocity, even in the absence of a sensor (gyroscope and/or accelerometer), but by the simple computerized visual analysis of the movement of the head.

The device calculates and modulates the display time considering that the diagnosis of obstructive sleep apnea syndrome has an average response and an average letter display time which has been estimated to be approximately 85% (but may vary with further research and data).

In a preferred embodiment, the recording unit (3) may be computer software.

The recording unit (3) is also able to record the response of the patient. In fact, once the patient has read and recognized the letter shown, he will communicate it to the clinician, who will introduce said result in the recording unit (3). Alternatively, the patient himself may introduce the response into the unit (2), for example, with the use of a keyboard in the preferred embodiment, wherein the recording unit (3) is computer software.

Optionally, the apparatus of the invention may further include an analysing unit (4). Said analysing unit is connected to the recording unit (3), so that it may acquire data on the patient's responses to be analysed, and may be connected to the measuring unit (2) or to the recording unit (3) in order to acquire data on the rotational acceleration value achieved.

In a preferred embodiment, the recording unit (3) and the analysing unit (4) may be represented by computer software.

The analysing unit (4) is also connected to the displaying unit (1), so that it receives information on the letter shown and compares it to the response given by the patient. In particular, when they match, the patient's response will be correct or positive, otherwise it will be wrong or null. The analysing unit (4) is able to analyse the collected results about rotational acceleration and patient's response in each set of experiments and to provide an output, which may be in any suitable form, such as signals, tables or graphs, like column graph, wherein the x and y axes report, for example, the value of the rotational acceleration and the frequency of positive results, respectively.

Consequently, the trend in frequency of positive results when rotational acceleration increases would thus become evident. In a further embodiment, the analysing unit (4) may provide an output in the form of signals, visual signal, for example through the displaying unit (1) or a sound signal, through any suitable means connected thereto, informing, for example, the clinician about the correctness of each response, by comparing the letter shown on the displaying unit (1) and the response of the patient.

According to a preferred embodiment, the displaying unit (1) may be a video, a mobile phone screen or a visualization video on virtual reality or augmented reality.

According to a preferred embodiment, the displaying unit (1) may be a video, a mobile phone screen or a visualization video on virtual reality or augmented reality of an image or a series of images.

According to a preferred embodiment, the head rotational acceleration measuring unit (2) is placed in such a way that it rotates contextually (integrally) with the patient's head.

According to a preferred embodiment, the head rotational acceleration measuring unit (2) may be constituted by a gyroscope and/or an accelerometer, or it may be an optical device for reading or a very high-speed camera which detects the movement of the head.

According to a preferred embodiment, the device further comprises an analysing unit (4).

According to a preferred embodiment, the displaying unit (1) may be a screen on which visual targets are displayed or projected.

According to a preferred embodiment, the displaying unit (1) may be a computer monitor.

According to a preferred embodiment, the recording unit (3) may be computer software.

According to a preferred embodiment, the recording unit (3) records the data on the rotational acceleration values and the patient's responses.

According to a preferred embodiment, the patient's response is introduced by the patient himself into the recording unit (3).

According to a preferred embodiment, the recording unit (3) is connected to the rotational acceleration measuring unit (2) and/or to the analysing unit (4).

According to a preferred embodiment, the analysing unit (4) analyses the collected results about the rotational acceleration and the patient's responses.

According to a preferred embodiment, the analysing unit (4) is connected to the displaying unit (1).

According to a preferred embodiment, the analysing unit (4) provides an output.

According to a more preferred embodiment, said output may be in the form of signals, tables or graphs.

According to a preferred embodiment, the treatment of obstructive sleep apnea syndrome, performed with the aforementioned device, may be used in the preventive and rehabilitative phase.

According to a preferred embodiment, the analysis of the functional level of the vestibule-ocular reflex (VOR) is integrated in the device, in particular in the analysing unit (4) to allow the development of the prevention and/or rehabilitation of the syndrome from obstructive sleep apnea.

According to a preferred embodiment, the functional level of the VOR of a subject has a deficit calculated by the aforementioned device at the time of the investigation of the active or passive impulsive head movement, it is comprised from 100 degrees/s² to 20,000 degrees/s² of peak acceleration of head movement, said movement being comprised from 2 degrees to 45 degrees, preferably about 10 degrees on average.

According to a preferred embodiment, the functional level of the VOR of a subject has a deficit calculated by the aforementioned device at the time of the investigation of the active or passive impulsive head movement, it is comprised from 10 degrees/s to 800 degrees/sec of peak velocity of the head movement, said movement being from 2 degrees to 45 degrees, preferably about 10 degrees on average.

According to a preferred embodiment, the measurement of the functional level of the VOR is carried out by displaying in the displaying unit (1) optokinetic confounding backgrounds and/or confounding videos (to increase the reading difficulty), in the preventive phase and/or in the rehabilitation phase of obstructive sleep apnea syndrome.

Another object is the use of the device for the treatment of obstructive sleep apnea syndrome or to improve physical recovery during the sleep phase or to improve night-time breathing and / or to reduce noise emission during sleep. It has in fact been found that the device of the invention allows to improve physical recovery during the sleep phase, also allowing to sleep better, and also to improve the night-time breathing and / or also to reduce noise emission during sleep, preferably of adults, including adults without obstructive sleep apnea syndrome.

According to a preferred embodiment, the device of the invention is used in combination with the device for the production of melatonin for rehabilitation from OSAS, to improve physical recovery during the sleep phase or to improve the night-time breathing. The device for the production of melatonin is described in the international application number WO2019087121A1 in the name of Beon Solutions S.r.l. published on May 9, 2019. In particular, said device for the production of melatonin is capable of generating light radiation in a length range comprised between 350 and 750 nm., preferably in the red region, i.e. from 625 to 740 nm.

Another object is a diagnostic method for identifying individuals potentially suitable for developing obstructive sleep apnea syndrome comprising the following steps:
a. to place the person to be diagnosed in front of the device described above and to display, through the displaying unit (1), moving letters;
b. by means of an operator, subject the person's head to passive movements in the direction of the semi-circular canals, wherein said movements are unpredictable and have an acceleration ranging from 300 to 18,000 degree /s² of acceleration peak on a head movement comprised from 5 to 20 degrees;
c. by means of the displaying unit (1) display the letter or optotype or combination of letters or words for a period of time ranging from 20 milliseconds to 700 milliseconds and to verify the correctness of the reading through a response from the person.
d. by means of the recording unit (3), to analyse the velocities and accelerations at which the person was unable to make the correct readings;
e. wherein the reading of the person is found to be correct below 85% of the positive responses of a single optotype to an acceleration between 300 and 7000 degrees/s² of maximum acceleration for an appearance time between 30 and 120 milliseconds, that person is potentially capable of developing obstructive sleep apnea syndrome.

It has in fact been found that an incorrect reading under 85% of positive responses of a single optotype at a velocity ranging from 300 to 7000 degrees/s² of maximum acceleration (the maximum velocity is equal acceleration divided by 19.2) for an appearance time between 30 and 120 milliseconds is closely correlated with the pathology of obstructive sleep apnea syndrome.

According to a preferred embodiment, step a) is performed according to the method following described, including the preferred embodiments.

During step a), the person is required, while sitting or standing, to read the letters of an optotypic line on a conveniently rescaled optotype chart projected on the displaying unit (1) placed in front of them, usually at a suitable distance comprised for example between 30 and 250 centimeters, preferably 150 centimeters, as shown in Fig. 1.

During step a), the person is required to read and recognize a letter shown to them, while the clinician rotates their head imparting a certain rotational acceleration (see, for a general reference, "Principle of the head impulse test (thrust) or Halmagyi head thrust test (HHTT)" F. Wuyts, B-ENT, 2008, 4, Suppl. 8, 23-25). In particular, with reference to Fig. 1, the person sits or stands in front of a displaying unit (1) at a distance between about 30 centimeters and about 150 centimeters, preferably being about 50 centimeters, wearing a helmet or a band which measures the velocity and acceleration of the head because of the acceleration measuring unit (2) which is attached to said helmet or band, so that said unit (2) moves contextually (integrally) with the patient's head.

During the execution of the test, the clinician stands, for example, behind the patient and poses his hands on the cranio-parietal bones. In each experiment the clinician rotates the patient's head to the left or to the right around its vertical axis, thus imposing a certain rotational acceleration. Said acceleration value is measured by the measuring unit (2) and may be recorded by the recording unit (3). When said reaches acceleration or overcomes a threshold value previously set by the clinician, the letter of a Sloan appears on the displaying unit (1). Said threshold value may for example determine an acceptable head acceleration rangel of 600 degrees/s²; for example, it may be set to about 1000 degrees/s², thereby defining a value between about 700 and about 1300 degrees/s² in the course of the first set of experiments, while during the subsequent set the head acceleration may achieve about 1,700-2,300 degrees/s².

In particular, with respect to the size of the Sloan's letter shown in said first experiments, this has the size of the letters of the optotypic line immediately superior to the ones read by the patient during the static acuity assessment phase of step a). For example, if the patient was able to read the letters of the ninth optotypic line, then during the dynamic test the letters belonging to the eighth line, having a larger size, are shown. As for the appearance time of the letter, it may be between 10-150 milliseconds and, preferably, between 25-95 milliseconds from the time when the acceleration of the head reaches the threshold. The person is required to read and recognize it and the response is then recorded in the recording unit (3).

According to a preferred embodiment, in step a) the person is required, while sitting or standing, to read the letters of an optotype line on an optotype table.

According to a preferred embodiment, in step a) said optotype graphic is placed in front of it at a distance comprised between about 30 and about 250 centimeters, and preferably about 150 centimeters.

According to a preferred embodiment, in step b) in each experiment of each set of experiments the person is required to actively rotate the head from left to right.

According to a preferred embodiment, when the rotational acceleration imparted by said active movements reaches a threshold value, a visual target appears on the displaying unit (1).

According to a preferred embodiment, said threshold value is set by the clinician or by the patient himself according to the instructions of the clinician in the analysing unit (4).

According to a preferred embodiment, in step b) each experiment is repeated for a number of times decided by the clinician based on his experience.

## Claims

1. Device for the treatment of obstructive sleep apnea syndrome comprising:
- a displaying unit (1),
- a head rotational acceleration measuring unit (2) and
- a recording unit (3).

2. Device according to claim 1, wherein the displaying unit (1) may be a video, a mobile phone screen or a visualization video on virtual reality or augmented reality.

3. Device according to any one of claims from 1 to 2, wherein the rotational acceleration measuring unit (2) may be constituted by a gyroscope and/or an accelerometer or by an optical device for reading the movement of the head which calculates it angular velocity with computerized visual analysis of head movement.

4. Device according to any one of claims from 1 to 3, further comprising an analysing unit (4).

5. Device according to claim 4, wherein the analysis of the functional level of the vestibule-ocular reflex (VOR) is integrated in the analysing unit (4).

6. Device according to claim 5, wherein the functional level of the VOR of a subject has a deficit calculated by the device according to any one of claims from 1 to 5 at the time of the investigation of the active or passive impulsive head movement, it is comprised from 100 degrees/s² to 20,000 degrees/s² of peak acceleration of the head movement, said movement being from 2 degrees to 45 degrees.

7. Device according to any one of claims from 5 to 6, wherein the functional level of the VOR of a subject has a deficit calculated by the device according to any of claims from 1 to 5 at the moment of the investigation of the active or passive impulsive head movement, it is comprised from 10 degrees/s to 800 degrees/sec of peak velocity of head movement, said movement being from 2 degrees to 45 degrees.

8. Device according to any of claims from 1 to 7, wherein the displaying unit (1) is suitable for displaying optokinetic confounding backgrounds and/or confounding videos.

9. Use of the device according to any one of claims from 1 to 8 for the treatment of obstructive sleep apnea syndrome or to improve physical recovery during the sleep phase or to improve night breathing and / or to reduce noise emission during sleep.

10. Use of the device according to claim 9, wherein said device is used in combination with the device for producing melatonin.

11. Diagnostic method for identifying individuals potentially suitable for developing obstructive sleep apnea syndrome comprising the following steps:
a. to place the person to be diagnosed in front of the device according to any of claims from 1 to 3 and to display, through the displaying unit (1), moving letters;
b. by means of an operator, subject the person's head to passive movements in the direction of the semi-circular canals, wherein said movements are unpredictable and have an acceleration ranging from 300 to 18,000 degrees/s² of acceleration peak on a head movement comprised from 5 to 20 degrees;
c. by means of the displaying unit (1) display the letter or optotype or combination of letters or words for a period of time ranging from 20 milliseconds to 700 milliseconds and to verify the correctness of the reading through a response from the person.
d. by means of the recording unit (3), to analyse the velocities and accelerations at which the person was unable to make the correct readings;
e. wherein the reading of the person is found to be correct below 85% of the positive responses of a single optotype to an acceleration between 300 and 7000 degrees/s² of maximum acceleration for an appearance time between 30 and 120 milliseconds, that person is potentially capable of developing obstructive sleep apnea syndrome.
